# FASCICULE DE BREVET EUROPEEN

(11) **EP 2 730 562 B1**
(45) Date de publication et mention de la délivrance du brevet: **25.03.2015**
(21) Numéro de dépôt: 13191850.0
(22) Date de dépôt: 07.11.2013
(51) Int. Cl.: C07D 223/16, C07C 253/30

(54) **Nouveau procédé de synthèse du (2E)-3-(3,4-dimethoxyphényl)prop-2-enenitrile, et application à la synthèse de l'ivabradine et de ses sels d'addition à un acide pharmaceutiquement acceptable**
Neues Syntheseverfahren von (2E)-3-(3,4-Dimethoxyphenyl)Prop-2-Enenitril, und Anwendung zur Synthese von Ivabradin und seinen Additionssalzen zu einer pharmazeutisch akzeptierbaren Säure
Novel method for synthesising (2E)-3-(3,4-dimethoxyphenyl)prop-2-enenitrile and use for synthesising ivabradine and the added salts thereof with a pharmaceutically acceptable acid

(30) Priorité: 08.11.2012 FR 1260576
(43) Date de publication de la demande: 14.05.2014
(73) Titulaire: Les Laboratoires Servier, 92284 Suresnes Cedex (FR)
(72) Inventeur: Carranza, Maria del Pilar, 13670 Villarrubia de los Ojos (ES); Garcia Aranda, Maria Isabel, 45006 Toledo (ES); Gonzalez, José Lorenzo, 45007 Toledo (ES); Sanchez, Frédéric, 45111 Cobisa (ES)

(56) Documents cités:
- EP-A1- 0 534 859
- WO-A1-2011/138625
- DE-A1- 2 303 919
- SPENCER A: "A HIGHLY EFFICIENT VERSION OF THE PALLADIUM-CATALYSED ARYLATION OF ALKENES WITH ARYL BROMIDES", JOURNAL OF ORGANOMETALLIC CHEMISTRY, ELSEVIER-SEQUOIA S.A. LAUSANNE, CH, vol. 258, 1 janvier 1983 (1983-01-01), pages 101-108, XP001205429, ISSN: 0022-328X, DOI: 10.1016/0022-328X(83)89511-4
- KAMETANI TETSUJI ET AL: "Syntheses of (+-)-tetrahydropalmatine and spirobenzylisoquinolines by thermolysis of benzocyclobutene derivatives", JOURNAL OF THE CHEMICAL SOCIETY, PERKIN TRANSACTIONS 1, CHEMICAL SOCIETY, LETCHWORTH; GB, no. 10, 1 janvier 1985 (1985-01-01), pages 2151-2154, XP008162620, ISSN: 0300-922X, DOI: 10.1039/P19850002151
- ZHAO SHENG YIN ET AL: "A practical synthesis of 1-cyano-4,5-dimethoxybenzocyclobutene", JOURNAL OF CHEMICAL RESEARCH, SCIENCE REVIEWS LTD, GB, no. 7, 1 janvier 2009 (2009-01-01), pages 420-422, XP008162622, ISSN: 0308-2342

## Description

La présente invention concerne un procédé de synthèse du (2*E*)-3-(3,4-diméthoxyphényl)prop-2-ènenitrile de formule (I) : 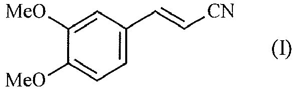 et son application à la synthèse de l'ivabradine et de ses sels d'addition à un acide pharmaceutiquement acceptable.

Le composé de formule (I) obtenu selon le procédé de l'invention est utile dans la synthèse de l'ivabradine de formule (II) : 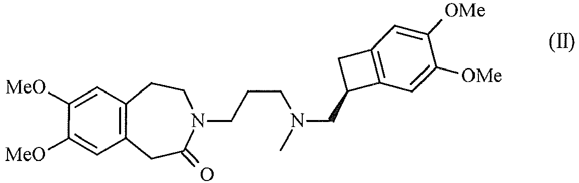 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
qui peut être transformée en un de ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique et en un de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrites dans le brevet européen EP 0 534 859.

Ce brevet décrit la préparation de l'ivabradine à partir du 3,4-diméthoxybicyclo[4.2.0]octa-1,3,5-triène-7-carbonitrile de formule (III) : 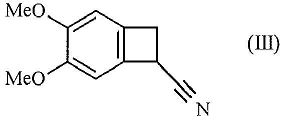 qui est transformé en composé de formule (IV) : 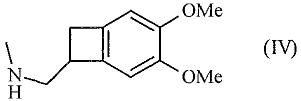 qui est dédoublé pour conduire au composé de formule (V) : 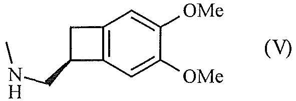 qui est mis en réaction avec le composé de formule (VI) : 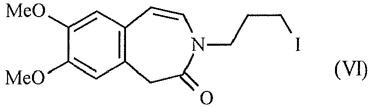 pour conduire au composé de formule (VII) : 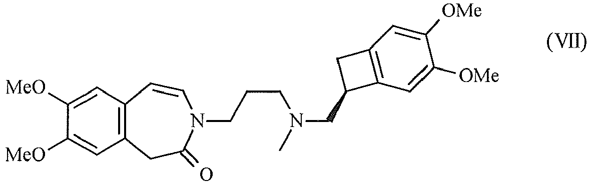 dont l'hydrogénation catalytique conduit à l'ivabradine, qui est alors transformée en son chlorhydrate.

La préparation du composé de formule (III) à partir du (3-(2-bromo-4,5-diméthoxyphényl)propanenitrile de formule (VIII) est décrite dans Tetrahedron 1973, 29, pp 73-76 :

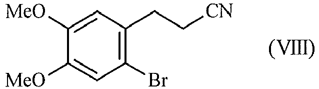

Le composé de formule (I), précurseur du composé de formule (VIII), est donc un intermédiaire clé dans la synthèse de l'ivabradine.

La demande de brevet DE 2 303 919 décrit la préparation du composé de formule (I) à partir du 3,4-diméthoxybenzaldéhyde avec un rendement de 74%.

Compte-tenu de l'intérêt industriel de l'ivabradine et de ses sels, il était impératif de trouver un procédé performant permettant d'accéder au (2*E*)-3-(3,4-diméthoxyphényl)prop-2-ènenitrile de formule (I) avec un excellent rendement.

La présente invention concerne un procédé de synthèse du composé de formule (I) : 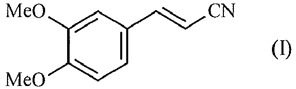 **caractérisé en ce que** le composé de formule (IX) : 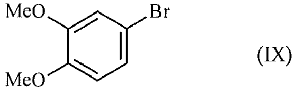 est soumis à une réaction de couplage avec l'acrylonitrile en présence d'un catalyseur au palladium, d'un ligand, d'une base et d'un agent de transfert de phase dans un solvant organique pour conduire au composé de formule (I).

Parmi les catalyseurs au palladium pouvant être utilisés pour effectuer la transformation du composé de formule (IX) en composé de formule (I), on peut citer à titre non limitatif le diacétate de palladium, le palladium sur charbon et le dichlorure de palladium.

Le catalyseur au palladium préférentiellement utilisé pour effectuer la transformation du composé de formule (IX) en composé de formule (I) est le palladium sur charbon.

Parmi les ligands pouvant être utilisés pour effectuer la transformation du composé de formule (IX) en composé de formule (I), on peut citer à titre non limitatif la triphénylphosphine et la tri(*o*-tolyl)phosphine.

Le ligand préférentiellement utilisé pour effectuer la transformation du composé de formule (IX) en composé de formule (I) est la tri(o-tolyl)phosphine.

Parmi les bases pouvant être utilisées pour effectuer la transformation du composé de formule (IX) en composé de formule (I), on peut citer à titre non limitatif la triéthylamine, l'acétate de sodium, le carbonate de sodium et le carbonate de potassium.

La base préférentiellement utilisée pour effectuer la transformation du composé de formule (IX) en composé de formule (I) est l'acétate de sodium.

Parmi les agents de transfert de phase pouvant être utilisés pour effectuer la transformation du composé de formule (IX) en composé de formule (I), on peut citer à titre non limitatif le bromure de tétrabutylammonium et le chlorure de tétrabutylammonium.

L'agent de transfert de phase préférentiellement utilisé pour effectuer la transformation du composé de formule (IX) en composé de formule (I) est le bromure de tétrabutylammonium.

Parmi les solvants organiques pouvant être utilisés pour effectuer la transformation du composé de formule (IX) en composé de formule (I), on peut citer à titre non limitatif le *N*,*N*-diméthylacétamide et le *N*,*N*-diméthylformamide.

Le solvant préférentiellement utilisé pour effectuer la transformation du composé de formule (IX) en composé de formule (I) est le *N*,*N*-diméthylacétamide.

La transformation du composé de formule (IX) en composé de formule (I) est conduite à une température préférentiellement comprise entre 100°C et 170°C.

La présente invention concerne également un procédé de synthèse du composé de formule (VIII) à partir du composé de formule (I), préparé selon le procédé décrit plus haut, **caractérisé en ce que** ledit composé de formule (I) : 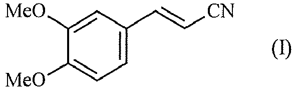 est transformé en composé de formule (X) : 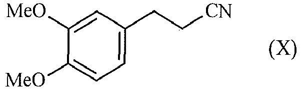 par une réaction de réduction,
lequel est transformé en composé de formule (VIII) : 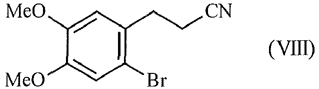 par une réaction de bromation.

La réaction de réduction du composé de formule (I) peut être réalisée dans les conditions décrites pour le dérivé bromé correspondant dans la demande de brevet CN 101 407 474 et dans la publication J Chem. Res. 2009 (7), 420-422.

La réaction de bromation du composé de formule (X) peut être réalisée dans les conditions décrites sur des composés semblables dans les publications J. Chem. Soc., Perkin Trans I 1985, 2151-2154 et J. Chem. Soc., Perkin Trans I 1991, 1749-1754.

La préparation du composé de formule (VIII) par une réaction de bromation du composé de formule (X) a également été décrite en présence de dibrome dans l'acide acétique dans J. Org. Chem 1972, vol. 37, n°21, pp 3374-3376, avec un rendement de 48%.

La présente invention concerne également un procédé de synthèse de l'ivabradine à partir du composé de formule (I) préparé selon le procédé de l'invention et transformé en composé de formule (VIII) selon l'enchaînement réactionnel décrit plus haut. Le composé de formule (VIII) est ensuite transformé en composé de formule (III) en suivant l'enseignement de l'art antérieur (Tetrahedron 1973, 29, pp 73-76) par une réaction de cyclisation intramoléculaire en milieu basique ; ledit composé de formule (III) étant ensuite transformé en ivabradine selon le procédé décrit dans EP 0 534 859.

Les exemples suivants illustrent l'invention.

Les points de fusion ont été mesurés avec un appareil BÜCHI Melting Point B-545 (Volt. 230VAC, Freq. 50/60 Hz, Power max. 220W).

### Liste des abréviations utilisées

DMAC : *N*,*N*-diméthylacétamide
P.F. : point de fusion
THF : tétrahydrofurane

### Exemple 1 : (2E)-3-(3,4-diméthoxyphényl)prop-2-ènenitrile

Un mélange de 5 g de 4-bromo-1,2-diméthoxybenzène (3,31 mL, 23 mmoles), 3,2 g d'acrylonitrile (3,9 mL, 60 mmoles, 2,6 éq.), 2,3 g d'acétate de sodium (27,6 mmoles, 1,2 éq.), 7,4 g de bromure de tétrabutylammonium (23 mmoles, 1 éq.), 0,7 g de tri(o-tolyl)phosphine (2,3 mmoles, 0,1 éq.) et 4,9 g de palladium 5% sur charbon (2,3 mmoles, 0,1 éq.) dans 25 mL de DMAC est préparé. La suspension noire est agitée à reflux pendant 12 heures. Le milieu réactionnel est ramené à température ambiante et filtrée. Le résidu solide est rincé deux fois au toluène. Les filtrats sont rassemblés et évaporés sous pression réduite. Le brut réactionnel est purifié sur colonne de silice (éluant : méthylcyclohexane : acétate d'éthyle 6 :4) pour conduire à 1.4 g du produit attendu.
Rendement = 33%
P.F.= 92-99°C

### Exemple 2 : 3-(3,4-diméthoxyphényl)propanenitrile

A une solution de 1 g (5,3 mmoles) de (2*E*)-3-(3,4-diméthoxyphényl)prop-2-ènenitrile dans 9,3 mL de pyridine et 2,8 mL de méthanol est ajouté petit à petit 0,24 g de NaBH₄ (6,3 mmol, 1,2 éq.). Le milieu réactionnel est chauffé à reflux pendant 9 heures. Après refroidissement à température ambiante, le milieu réactionnel est additionné à une solution de 9 mL d'acide chlorhydrique à 37% dans 24 g de glace. La solution est extraite deux fois au dichlorométhane. Les phases organiques sont rassemblées et le solvant est évaporé sous pression réduite pour conduire à 0,82 g d'une huile brun rouge qui cristallise.
Rendement = 82%
P.F. = 47-48°C

### Exemple 3 : 3-(2-bromo-4,5-diméthoxyphényl)propanenitrile

Le composé du titre est préparé d'après le mode opératoire décrit dans la publication J. Chem. Soc., Perkin Trans I 1985, 2151-2154 pour la préparation du 3-(2-bromo-5,6-diméthoxyphényl)propanenitrile) :
A un mélange de 21 g 3-(3,4-diméthoxyphényl)propanenitrile, 10,3 g d'acétate de sodium et 400 mL d'acide acétique, sont ajoutés 20g de dibrome dans 50 mL d'acide acétique. Le milieu réactionnel est ainsi agité pendant une nuit puis versé dans l'eau et extrait au benzène. La phase organique est lavée avec une solution aqueuse de thiosulfate de sodium puis avec de l'eau, séchée sur sulfate de sodium et concentrée sous pression réduite. Le brut réactionnel est purifié sur colonne de silice (éluant : benzène), le produit obtenu est recristallisé dans l'éthanol pour conduire à 19,3 g du produit attendu.
Rendement = 65%
P.F. : 78-80°C

### Exemple 4 : 3,4-diméthoxybicyclo[4.2.0]octa-1,3,5-triène-7-carbonitrile

D'après Tetrahedron 1973, 29, pp 73-76

A une solution de NaNH₂, préparée à partir de 200 mL de NH₃ liquide et d'1 g de Na (catalyseur FeCl₃), sont ajoutés par portions 5,4 g de 3-(2-bromo-4,5-diméthoxyphényl)propanenitrile et le mélange réactionnel est agité à température ambiante pendant 2 heures. Après évaporation de l'excès de NH₃, 2 g de NH₄Cl et 200 mL d'eau sont ajoutés par portions. Les cristaux gris formés sont collectés et recristallisés dans l'éthanol pour conduire à 2,38 g du produit attendu
Rendement = 74%
P.F. = 84-85°C

### Exemple 5 : 3,4-diméthoxy-N-méthylbicyclo[4.2.0]octa-1,3,5-trién-7-amine

D'après EP 0 534 859

### Stade 1 : chlorhydrate de 3,4-diméthoxybicyclo[4.2.0]octa-1,3,5-trién-7-amine

On ajoute goutte à goutte et sous agitation à température ambiante, 312 mL d'une solution molaire de borane complexé avec du THF à une solution de 25 g de 3,4-diméthoxybicyclo[4.2.0]octa-1,3,5-triène-7-carbonitrile dans 250 mL de THF. On laisse en contact pendant 12 heures, puis on ajoute 200 mL d'éthanol et on agite 1 heure. On ajoute, goutte à goutte, 100 mL d'éther chlorhydrique 3,3N. On obtient 27,7 g du produit attendu.
Rendement = 90%
P.F. = 205°C

### Stade 2 : (3,4-diméthoxybicyclo[4.2.0]octa-1,3,5-trién-7-yl)carbamate d'éthyle

On coule 1,5 mL de chloroformiate d'éthyle sur une suspension de 3,4 g du composé obtenu au stade 1 de 4,5 mL de triéthylamine et de 50 mL de dichlorométhane. On laisse une nuit sous agitation à température ambiante puis on lave à l'eau et à l'acide chlorhydrique 1N. On sèche et on évapore à sec le solvant. On obtient 3,2 g d'une huile correspondant au produit attendu.
Rendement = 80%

### Stade 3 : 3,4-diméthoxy-N-méthylbicyclo[4.2.0]octa-1,3,5-trién-7-amine

On ajoute 3,2 g du composé obtenu au stade 2 en solution dans 30 mL de THF à une suspension de 0,9 g de LiAlH₄ dans 20 mL de THF. On porte au reflux 1 heure 30 minutes puis on hydrolyse par 0,6 ml d'eau et 0,5 mL de soude à 20%, et enfin par 2,3 mL d'eau. Les sels minéraux sont ensuite filtrés, rincés au THF puis le filtrat obtenu est évaporé à sec. On obtient 2,3 g du composé attendu.
Rendement = 92%

### Exemple 6 : (7S)-3,4-diméthoxy-N-méthylbicyclo[4.2.0]octa-1,3,5-trién-7-amine

D'après EP 0 534 859

On fait réagir la 3,4-diméthoxy-*N*-méthylbicyclo[4.2.0]octa-1,3,5-trién-7-amine avec une quantité équimolaire de l'acide (d) camphosulfonique dans l'éthanol. Après évaporation sous vide du solvant, le sel est recristallisé une première fois dans l'acétate d'éthyle puis dans l'acétonitrile jusqu'à l'obtention de l'énantiomère cible avec une pureté optique supérieure à 99% (évaluation par HPLC sur colonne Chiralcel ^{®} OD).

### Exemple 7 : 3-{3-[{[(7S)-3,4-diméthoxybicyclo[4.2.0]octa-1,3,5-trién-7-yl]méthyl}(méthyl)amino]propyl}-7,8-diméthoxy-1,3-dihydro-2H-3-benzazépin-2-one

D'après EP 0 534 859

La solution de sel de (d) camphosulfonate obtenu à l'exemple 6 dans l'acétate d'éthyle est amenée à pH basique à l'aide d'hydroxyde de sodium puis la phase organique est séparée, lavée, séchée sue Na₂SO₄ et évaporée.

On porte ensuite au reflux pendant 18 heures un mélange composé de 5,6 g de carbonate de potassium, 2,2 g de l'amine ci-dessus dans 100 mL d'acétone et de 4g de 3-(3-iodopropyl)-7,8-diméthoxy-1,3-dihydro-2*H*-3-benzazépin-2-one.

On évapore le solvant sous vide, on reprend le résidu à l'acétate d'éthyle, puis on extrait à l'acide chlorhydrique 3N.

On amène la phase aqueuse décantée à pH basique à l'aide d'hydroxyde de sodium, puis on l'extrait à l'acétate d'éthyle. Après lavage à neutralité et séchage sur MgSO₄, on évapore sous vide pour obtenir 4,5 g d'une huile qui est purifiée sur colonne de silice en utilisant comme éluant un mélange dichlorométhane/méthanol (90/10).
Rendement = 64%

### Exemple 8 : 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

D'après EP 0 534 859

5 g de 3-{3-[{[(7S)-3,4-diméthoxybicyclo[4.2.0]octa-1,3,5-trién-7-yl]méthyl}(méthyl)amino]propyl}-7,8-diméthoxy-1,3-dihydro-2*H*-3-benzazépin-2-one dans 50 mL d'acide acétique glacial sont hydrogénés dans un appareil de Parr, sous un pression de 4,9 bar d'hydrogène à température ambiante pendant 24 heures, en présence d'1 g d'hydroxyde de palladium à 10%. On filtre le catalyseur, on évapore le solvant, puis on reprend le résidu sec à l'eau et à l'acétate d'éthyle. On sèche la phase organique sur sulfate de magnésium anhydre, on concentre sous vide puis le résidu est purifié sur colonne de silice en utilisant comme éluant un mélange dichlorométhane/méthanol (95/5).

Après recristallisation dans l'acétate d'éthyle, on obtient 2g du composé attendu.
Rendement = 40%
P.F. = 101-103°C

## Revendications

1. Procédé de synthèse du composé de formule (I) : 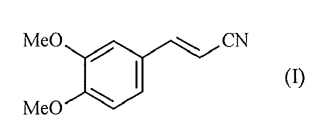 **caractérisé en ce que** le composé de formule (IX) : 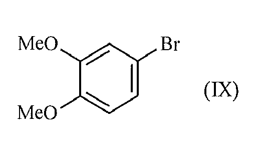 est soumis à une réaction de couplage avec l'acrylonitrile en présence d'un catalyseur au palladium, d'un ligand, d'une base et d'un agent de transfert de phase dans un solvant organique pour conduire au composé de formule (I).

2. Procédé selon la revendication 1, **caractérisé en ce que** le catalyseur au palladium utilisé pour effectuer la transformation du composé de formule (IX) en composé de formule (I) est choisi parmi le diacétate de palladium, le palladium sur charbon et le dichlourure de palladium.

3. Procédé selon la revendication 2, **caractérisé en ce que** le catalyseur au palladium utilisé pour effectuer la transformation du composé de formule (IX) en composé de formule (I) est le palladium sur charbon.

4. Procédé selon l'une quelconque des revendications 1 à 3, **caractérisé en ce que** le ligand utilisé pour effectuer la transformation du composé de formule (IX) en composé de formule (I) est choisi parmi la triphénylphosphine et la tri(o-tolyl)phosphine.

5. Procédé selon la revendication 4, **caractérisé en ce que** le ligand utilisé pour effectuer la transformation du composé de formule (IX) en composé de formule (I) est la tri-(o-tolyl)phosphine.

6. Procédé selon l'une quelconque des revendications 1 à 5, **caractérisé en ce que** la base utilisée pour effectuer la transformation du composé de formule (IX) en composé de formule (I) est choisie parmi la triéthylamine, l'acétate de sodium, le carbonate de sodium et le carbonate de potassium.

7. Procédé selon la revendication 6, **caractérisé en ce que** la base utilisée pour effectuer la transformation du composé de formule (IX) en composé de formule (I) est l'acétate de sodium.

8. Procédé selon l'une quelconque des revendications 1 à 7, **caractérisé en ce que** l'agent de transfert de phase utilisé pour effectuer la transformation du composé de formule (IX) en composé de formule (I) est choisi parmi le bromure de tétrabutylammonium et le chlorure de tétrabutylammonium.

9. Procédé selon la revendication 8, **caractérisé en ce que** l'agent de transfert de phase utilisé pour effectuer la transformation du composé de formule (IX) en composé de formule (I) est le bromure de tétrabutylammonium.

10. Procédé selon l'une quelconque des revendications 1 à 9, **caractérisé en ce que** le solvant organique utilisé pour effectuer la transformation du composé de formule (IX) en composé de formule (I) est choisi parmi le *N*,*N*-diméthylacétamide et le *N*,*N* diméthylformamide.

11. Procédé selon la revendication 10, **caractérisé en ce que** le solvant organique utilisé pour effectuer la transformation du composé de formule (IX) en composé de formule (I) est le *N*,*N*-diméthylacétamide.

12. Procédé selon l'une quelconque des revendications 1 à 11, **caractérisé en ce que** la transformation du composé de formule (IX) en composé de formule (I) est conduite à une température comprise entre 100°C et 170°C.

13. Procédé selon la revendication 1, **caractérisé en ce que** le composé de formule (I) obtenu est ensuite transformé en composé de formule (X) : 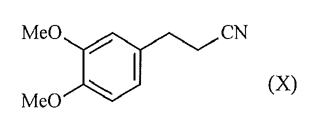 par une réaction de réduction,
lequel est transformé en composé de formule (VIII) : 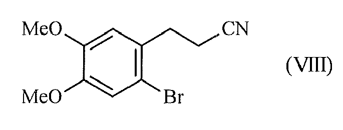 par une réaction de bromation.

14. Procédé de synthèse de l'ivabradine, de ses sels pharmaceutiquement acceptables et de ses hydrates, **caractérisé en ce** que :
- le composé de formule (IX) est transformé en composé de formule (I) selon le procédé de la revendication 1,
- le composé de formule (I) est ensuite transformé en composé de formule (VIII) selon le procédé de la revendication 13,
- le composé de formule (VIII) est ensuite transformé en ivabradine, qui peut être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

## Patentansprüche

1. Verfahren zur Synthese der Verbindung der Formel (I): 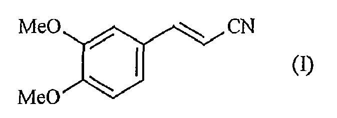 **dadurch gekennzeichnet, dass** die Verbindung der Formel (IX): 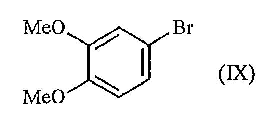 einer Kupplungsreaktion mit Acrylnitril in Gegenwart eines Palladiumkatalysators, eines Liganden, einer Base und eines Phasentransfermittels in einem organischen Lösungsmittel unterworfen wird zur Bildung der Verbindung der Formel (I).

2. Verfahren nach Anspruch 1, **dadurch gekennzeichnet, dass** der zur Durchführung der Umwandlung der Verbindung der Formel (IX) in die Verbindung der Formel (I) verwendete Palladiumkatalysator aus Palladiumdiacetat, Palladium-auf-Kohlenstoff und Palladiumdichlorid ausgewählt wird.

3. Verfahren nach Anspruch 2, **dadurch gekennzeichnet, dass** der zur Durchführung der Umwandlung der Verbindung der Formel (IX) in die Verbindung der Formel (I) verwendete Palladiumkatalysator Palladium-auf-Kohlenstoff ist.

4. Verfahren nach einem der Ansprüche 1 bis 3, **dadurch gekennzeichnet, dass** der zur Durchführung der Umwandlung der Verbindung der Formel (IX) in die Verbindung der Formel (I) verwendete Ligand aus Triphenylphosphin und Tri-(o-tolyl)-phosphin ausgewählt wird.

5. Verfahren nach Anspruch 4, **dadurch gekennzeichnet, dass** der zur Durchführung der Umwandlung der Verbindung der Formel (IX) in die Verbindung der Formel (I) verwendete Ligand Tri-(o-tolyl)-phosphin ist.

6. Verfahren nach einem der Ansprüche 1 bis 5, **dadurch gekennzeichnet, dass** die zur Durchführung der Umwandlung der Verbindung der Formel (IX) in die Verbindung der Formel (I) verwendete Base aus Triethylamin, Natriumacetat, Natriumcarbonat und Kaliumcarbonat ausgewählt wird.

7. Verfahren nach Anspruch 6, **dadurch gekennzeichnet, dass** die zur Durchführung der Umwandlung der Verbindung der Formel (IX) in die Verbindung der Formel (I) verwendete Base Natriumacetat ist.

8. Verfahren nach einem der Ansprüche 1 bis 7, **dadurch gekennzeichnet, dass** das zur Durchführung der Umwandlung der Verbindung (IX) in die Verbindung der Formel (IX) verwendete Phasentransfermittel aus Tetrabutylammoniumbromid und Tetrabutylammoniumchlorid ausgewählt wird.

9. Verfahren nach Anspruch 8, **dadurch gekennzeichnet, dass** das zur Durchführung der Umwandlung der Verbindung der Formel (IX) in die Verbindung der Formel (I) verwendete Phasentransfermittel Tetrabutylammoniumbromid ist.

10. Verfahren nach einem der Ansprüche 1 bis 9, **dadurch gekennzeichnet, dass** das zur Durchführung der Umwandlung der Verbindung der Formel (IX) in die Verbindung der Formel (I) verwendete organische Lösungsmittel aus N,N-Dimethylacetamid und N,N-Dimethylformamid ausgewählt wird.

11. Verfahren nach Anspruch 10, **dadurch gekennzeichnet, dass** das zur Durchführung der Umwandlung der Verbindung der Formel (IX) in die Verbindung der Formel (I) verwendete organische Lösungsmittel N,N-Dimethylacetamid ist.

12. Verfahren nach einem der Ansprüche 1 bis 11, **dadurch gekennzeichnet, dass** die Umwandlung der Verbindung der Formel (IX) in die Verbindung der Formel (I) bei einer Temperatur zwischen 100 °C und 170 °C durchgeführt wird.

13. Verfahren nach Anspruch 1, **dadurch gekennzeichnet, dass** die erhaltene Verbindung der Formel (I) anschließend durch eine Reduktionsreaktion in die Verbindung der Formel (X) umgewandelt wird: 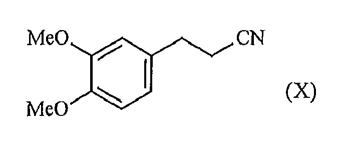 welche durch eine Bromierungsreaktion in die Verbindung der Formel (VIII) umgewandelt wird:

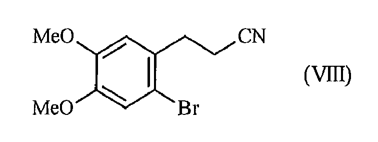

14. Verfahren zur Synthese von Ivabradin, seinen pharmazeutisch annehmbaren Salzen und seinen Hydraten, **dadurch gekennzeichnet, dass**:
- die Verbindung der Formel (IX) nach dem Verfahren von Anspruch 1 in die Verbindung der Formel (I) umgewandelt wird,
- die Verbindung der Formel (I) anschließend nach dem Verfahren von Anspruch 13 3 in die Verbindung der Formel (VIII) umgewandelt wird,
- die Verbindung der Formel (VIII) anschließend in Ivabradin umgewandelt wird, welches in seine Additionssalze mit einer pharmazeutisch annehmbaren Säure ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure und in ihre Hydrate umgewandelt wird.

## Claims

1. Process for the synthesis of the compound of formula (I): 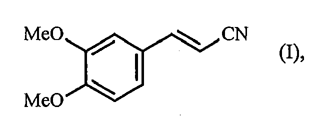 **characterised in that** the compound of formula (IX): 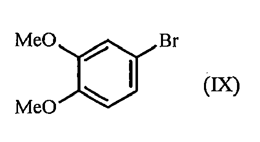 is subjected to a coupling reaction with acrylonitrile in the presence of a palladium catalyst, a ligand, a base and a phase transfer agent in an organic solvent to yield the compound of formula (I).

2. Process according to claim 1, **characterised in that** the palladium catalyst used to carry out the conversion of the compound of formula (IX) into the compound of formula (I) is selected from palladium diacetate, palladium on carbon, and palladium dichloride.

3. Process according to claim 2, **characterised in that** the palladium catalyst used to carry out the conversion of the compound of formula (IX) into the compound of formula (I) is palladium on carbon.

4. Process according to any one of claims 1 to 3, **characterised in that** the ligand used to carry out the conversion of the compound of formula (IX) into the compound of formula (I) is selected from triphenylphosphine and tri(o-tolyl)phosphine.

5. Process according to claim 4, **characterised in that** the ligand used to carry out the conversion of the compound of formula (IX) into the compound of formula (I) is tri(o-tolyl)phosphine.

6. Process according to any one of claims 1 to 5, **characterised in that** the base used to carry out the conversion of the compound of formula (IX) into the compound of formula (I) is selected from triethylamine, sodium acetate, sodium carbonate and potassium carbonate.

7. Process according to claim 6, **characterised in that** the base used to carry out the conversion of the compound of formula (IX) into the compound of formula (I) is sodium acetate.

8. Process according to any one of claims 1 to 7, **characterised in that** the phase transfer agent used to carry out the conversion of the compound of formula (IX) into the compound of formula (I) is selected from tetrabutylammonium bromide and tetrabutylammonium chloride.

9. Process according to claim 8, **characterised in that** the phase transfer agent used to carry out the conversion of the compound of formula (IX) into the compound of formula (I) is tetrabutylammonium bromide.

10. Process according to any one of claims 1 to 9, **characterised in that** the organic solvent used to carry out the conversion of the compound of formula (IX) into the compound of formula (I) is selected from *N*,*N*-dimethylacetamide and *N,N-*dimethylformamide.

11. Process according to claim 10, **characterised in that** the organic solvent used to carry out the conversion of the compound of formula (IX) into the compound of formula (I) is *N*,*N*-dimethylacetamide.

12. Process according to any one of claims 1 to 11, **characterised in that** the conversion of the compound of formula (IX) into the compound of formula (I) is carried out at a temperature between 100°C and 170°C, inclusive.

13. Process according to claim 1, **characterised in that** the compound of formula (I) obtained is subsequently converted into the compound of formula (X): 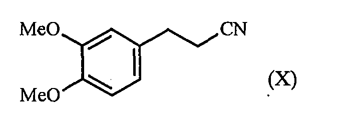 by a reduction reaction,
which compound is converted into the compound of formula (VIII): 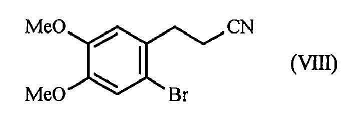 by a bromination reaction.

14. Process for the synthesis of ivabradine, pharmaceutically acceptable salts thereof and hydrates thereof, **characterised in that**:
- the compound of formula (IX) is converted into the compound of formula (I) in accordance with the process of claim 1,
- the compound of formula (I) is subsequently converted into the compound of formula (VIII) in accordance with the process of claim 13,
- the compound of formula (VIII) is subsequently converted into ivabradine, which may be converted into addition salts thereof with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into hydrates thereof.
